# EUROPEAN PATENT SPECIFICATION

(11) **EP 4 558 105 B1**
(45) Date of publication and mention of the grant of the patent: **03.06.2026**
(21) Application number: 23742153.2
(22) Date of filing: 03.07.2023
(51) Int. Cl.: A61F 5/02

(54) **ADJUSTABLE THERAPEUTIC APPARATUS FOR THE HUMAN TORSO**
VERSTELLBARER THERAPEUTISCHER APPARAT FÜR DEN MENSCHLICHEN TORSO
APPAREIL THÉRAPEUTIQUE RÉGLABLE POUR LE TORSE HUMAIN

(30) Priority: 18.07.2022 LV 220065
(43) Date of publication of application: 28.05.2025
(73) Proprietor: AGM ORTHOPAEDICS, SIA, 1055 Riga (LV)
(72) Inventor: MERTENS, Agris, 1055 Riga (LV)
(74) Representative: Kromanis, Artis
(86) International application number: PCT/LV2023/050011
(87) International publication number: WO 2024/019607

(56) References cited:
- FR-A1- 3 001 125
- RU-C2- 2 175 219
- US-A- 5 290 307
- US-A1- 2004 055 076
- US-A1- 2008 262 536
- US-A1- 2012 117 702
- US-B2- 10 898 365

## Description

### Field of the Invention

The present invention relates to adjustable therapeutic apparatus for the human torso.

### Background of the Invention

In the modern world, most of the day our spine is in the wrong position, which means that all organs and systems of the body are subjected to stress. And physical activity aimed at maintaining the muscles of the back is minimal. All these factors affect the development of osteoporosis and curvatures in the spine. Statistics indicate 80% of people who suffer from back pain during their lifetime. Back pain is not only a medical problem, but also a social and economic one. In terms of the number of days of disability, patients with back pain are among the leaders. If we turn to the risk factors for musculoskeletal pain associated with the working environment, they can be divided into four groups: individual, physical, clinical and psychological. Among the physical factors is a prolonged static position of the back, neck and arm, which leads to muscle spasm in the subscapular region, neck muscles and lower back. Also, they include stereotyping movements, non-compliance with ergometric conditions in the workplace, prolonged sedentary work and uncomfortable posture.

Prevention of diseases of the spine is in constant physical education. Physical exercises strengthen the muscles of the whole body, increase their strength, and also normalize the functions of the body. Also, an integral part of treatment and prevention is medical massage to relax tense areas of the back. An auxiliary tool for the treatment and restoration of the spine are orthopaedic corsets. Hard and semi-rigid. A corset is an auxiliary device that fixes, stabilizes and unloads the spine. This type of orthosis normalizes muscle tone and forms a physiologically correct position of the body, which helps relieve muscle spasm and back pain. Corsets are designed for the treatment and prevention of diseases of the spine.

Most of the existing braces and corsets have mainly the effect of rigidly stabilizing the human torso and providing low back mobility due to rigid materials and a strong girth. This negatively affects blood circulation in the skin and muscles in the corset area, which leads to a decrease in the effectiveness of treatment or, in some cases, to side complications.

The prior art discloses various therapeutic apparatuses. The US patent application publication No. US 4622957 discloses a therapeutic corset which is adapted for the sacrum, lumbar and thoracic region of the body comprises an elongated support surface formed from material that is bendable when subjected to forces encountered thereby. Disposed on the support surface and secured thereto along its edges is a flexible cover disposed thereover to form an envelope. The envelope is provided with a duct of introducing fluid therein and retaining same, thus inflating the envelope causing the surface to bend to assume a predetermined curvature, which is preferably in the shape of a crescent.

The US patent application publication No. US2021/0113359 discloses a spinal orthosis comprising an elongate and substantially rigid spinal frame having upper, middle and lower portions, and a lumbar assembly connected to the lower portion of the spinal frame.

The international application publication No. WO2009/052031 discloses an adjustable posterior spinal orthosis comprising a pair of flexible strap members removably attached at the top of the spinal member and secured and anchored at the bottom. Flexible straps form not only the shoulder straps but also contribute an adjustably fastening arrangement of smaller loops with connecting straps to abdominal pads. The abdominal pads can be fastened together and fastening pads can both secure and tighten the brace on the patient by pulling the flexible straps. French patent application publication No. FR3001125 discloses a clothing that has an elastic sheath intended to enclose a part of the trunk of a user, where the sheath includes an outer face and an inner face. An array of wooden balls is fixed at the inner face of the sheath such that array of balls rests directly against the enclosed part of the user when the garment is worn by the user. Each ball includes a central opening for passing ligaments, so as to form a chain of balls and to fix the chain of balls at the inner face of the sheath, where the ligaments are arranged so that the balls are movable in rotation. The US patent publication No. US5290307 discloses an acupressure belt for the treatment of lower back pain comprising a central fabric panel to which opposed elastic panels are affixed. The elastic panels terminate in a pair of end panels which may be joined together to retain the belt about the torso of the wearer. Located on the inner surface of the central panel is a plurality of acupressure-applying protrusions, each of which are individually positionable on the panel. The protrusions are positioned to apply pressure to the acupressure points associated with the lower spine.

### Summary of the Invention

The therapeutic apparatus for a human's torso differs from the prior art in that it can provide not only strengthening of the musculoskeletal system of the back, but also provides a relaxing effect like a medical massage. This is ensured by a combination of technical features of the invention located in anatomically correct places.

The apparatus has an inner side directed to the human's torso, and outer side directed outside from the human's torso. The apparatus comprises at least two acupressure elements with 1 mm to 10 cm distance between/from each other, wherein the acupressure elements are made of rigid material and comprising at least one canal therethrough for threading at least one string through the canal. The apparatus comprises at least two strings, which are an elongated elements made of a stretch material. The strings wrap the human's torso, and thus the strings press the acupressure elements towards the human's torso. The strings are arranged transversally in relation to the sagittal plane of the human. The apparatus comprises at least two string fasteners. Each of two ends of the string is fastened to the string fastener. One string fastener is attached to another string fastener, so that one string fastener overlaps another string fastener and thus changes the distance between the ends of the strings, and thus changes the tension of the strings. The string fasteners may have hook and loop or hook and eye, or rivet snaps. The apparatus comprises at least two reinforcing elements, which are elongated elements made from elastic and rigid material. Such materials elements are elastic in one direction and rigid in another direction. Typical example could be a plastic filament or rod. The reinforcing elements are arranged transversally in relation to the strings forming web like structure.

The therapeutic apparatus further comprises a back member, which is made of woven material at least from one layer of fabric and covers part of the back and lateral sides of human's torso. The back member comprises back member eyelets allowing the strings to be threaded therethrough. The apparatus further comprises at least one strap, which is an elongated element made of elastic material, which are continuation of the back member, extended from the back member in left direction and symmetrically to the sagittal plane the same numbers of straps extended from the back member in the right direction and straps wrap the human's torso. At each distal end from the back member the strap comprises a strap fastener. The reinforcing elements are associated with the back member so that the back member extends along all length of the reinforcing elements.

The back member may comprise a dividing cloth with dividing cloth eyelets, through which the strings are threaded as follows: the string is threaded from the outer side through the dividing cloth eyelet of the dividing cloth, then threaded through the acupressure element, then threaded through the dividing cloth eyelet of the dividing cloth from the inner side, and then threaded through the dividing cloth eyelet from the outer side of dividing cloth and so on alternates with at least two acupressure elements. In another embodiment the back member may comprise at least two layers of cloth, between which the acupressure elements, the reinforcing elements, the strings and the dividing cloth are positioned.

The apparatus may comprise a back string, where one end of the back string is attached to the back member and another end of the back string is attached to the back string fastener. The apparatus may comprise at least two loops located on the surface of the straps from outer side for threading the strings through it. The strings wrap around the human's back and are threaded through the loops for comfortable use of the therapeutic apparatus, so that even when the strings are unfastened, the fasteners remain in the part of the torso accessible to the human using the therapeutic apparatus. At least one of the strings or the back strings may have at least two sections with different elasticity, in result of which a different stroke length of the acupressure elements is provided when the strings and/or the back strings are stretched.

The therapeutic apparatus may further comprise at least two attachment points for attachment of the strap fasteners or the string fastener, which are located on the surface of straps or on a surface of the back member symmetrically to the sagittal plane. The strings and back strings that wrap around the human's torso may be threaded through the loops and, having made a 180-degree bend, are fastened to the attachment point at lateral or rear side of the back member by the string fastener or back string fastener.

In another embodiment of the invention at least one end of the one back string is fixed to the back member in a one place, and the other end of the back string is fixed to the back member in the other place, thus the back strings are stretched with the back member, when the straps are fastened around the human's torso.

The therapeutic apparatus may further comprise at least two cells for acupressure elements in the back member, formed by stitching together at least two fabric layers of the back member around the acupressure element with the string threaded through the acupressure element. At least two acupressure elements are located inside the cells for acupressure elements, thus the cells for acupressure elements limits the length of dislocation of acupressure element at length equal to acupressure element length, which provides a massage effect when the human moves using the apparatus.

The therapeutic apparatus may further comprise a pair of shoulder straps extending parallel and symmetrically to the sagittal plane from the upper part of the back member, and wrap around the human's shoulders and, using strap fasteners at the ends of the shoulder straps, are attached to the attachment point on the straps. The therapeutic apparatus may further comprise at least two strap lugs, which located on the surface of the strap, and a pair of the shoulder straps extending parallel and symmetrically to the sagittal plane from the upper part of the back member, wrap around the user's shoulders, are threaded through the strap lugs on the straps and, having made a 180-degree bend, are fastened on the attachment point on the shoulder strap by the strap fasteners on the shoulder straps. A pair of shoulder straps extend parallel and symmetrically to the sagittal plane from the upper part of the back member and wrap around the human's shoulders, and wherein shoulder straps are attached to the straps by the at least one elastic band.

The apparatus relieves tension in the back muscles, increases blood microcirculation and relieves back pain. The brace helps to restore the correct posture by mechanically increasing the curvature of the back waist - for this purpose, the sides of the apparatus have a specially designed tensioning mechanism, which, when tightened, allows to apply the curvature of the back regions that is suitable for different human torsos.

The apparatus adapts to any curvature of the body and even during intense movements, such as sports, the designated technical features of the apparatus fully occupies the required position.

The fabric of the apparatus is selected from the light and breathable materials and at the appropriately chosen tightening intensity, in result of which it will not cause any friction or body imprint.

The apparatus has several sectors of acupressure elements, which are arranged in a certain order in a mirror image relative to the sagittal plane of the human's torso. The distribution of acupressure elements interacts with the human's muscular corset thereby stimulates blood flow to the damaged areas of the spinal column. Zones of acupressure elements create a restorative effect. This effect is achieved by the fact that the pressure created by the acupressure elements is a completely new and unusual sensation for the human in the area of torso. The human's muscular corset is stimulated and activated for rehabilitation removing the load from the spine.

The apparatus has embodiments for the Sacro-lumbar, lumbar-thoracic and thoracic spine as interacts with torso lateral parts. The design of the apparatus includes acupressure elements that interact with the strings and are located in special cells that determine the location of the acupressure elements. Strings are located in several levels and can have both horizontal and vertical direction. Reinforcing elements separate the zones in which the acupressure elements are located. The reinforcing elements provide the apparatus shape and prevent the apparatus from bulging while wearing. The inner side of the apparatus fits snugly to the human's torso. The pressure created by the string conjunction with the acupressure elements does not create pain, and is selected so that it is not weak. Strings, divided into zones, and have adjustable fastening elements on the ends which greatly expands the possibilities of adjusting tension and fastening of the apparatus.

Acupressure elements used in the apparatus can be variable size and form. In another apparatus embodiment different size and form acupressure elements can be used together. Acupressure elements in the different size and shape can be used in the apparatus embodiment which will provide different acupressure effect to the different human's torso parts. In another embodiment the strings go through the canals in the acupressure elements so the acupressure element can slide on the string. In another embodiment the acupressure elements can be bonded with the strings. The acupressure elements can comprise a threaded hole arranged perpendicular to the canal for a set screw, allowing the set screw to hold the string within the canal thereby holding the acupressure element from sliding along the string. Number of the canals can be as one as many as strings goes through the acupressure element. In another embodiment of the acupressure element and the string combination, attachment of the acupressure element to the string can be done through the acupressure element surface.

Acupressure elements on parallelly located strings can be arranged symmetrically parallel to each other, as well as in other positions. The apparatus is divided into sectors that allow the formation of independent pressure on various elements of the human's torso. By changing the tension force of the strings, pressure of the acupressure elements to the body also changes. The principle of work of the sectors allows to act on different muscle groups, thereby helping to give the best therapeutic effect.

In various apparatus embodiments one or more strings can be used. Each string can carry one or more acupressure elements. String tension load can be uniform or variable through the all-element body. This provides the possibility to create different tensile load between the acupressure elements. Also, variable and uniform stretching load strings can be used in one apparatus design. Apparatus embodiments enclose strings in different directions like parallel, diagonal or crossing. Independently of placement of the tension elements, they can be attached to the apparatus each separately or bonded together to the one point in the apparatus inside constructions, outer or inner surface. If there is a single point connection of the strings the length of the string element can be different or similar. Pulling of the fastening element of the string in the embodiment of different lengths provides different tension load in the elements and pressure created by the acupressure elements. In the case of spinal correction, strings of various lengths increase the lordosis of the spine and enhance the effect of acupressure on certain parts of the human's back. Through the length the string it can be formed from non-stretchable elements and elastic or semi elastic regions.

The string fastener can be attached to the attachment point of apparatus or attached with other string fastener.

Strings can be bonded to the back member of the apparatus. The string one point can be fixed to the brace or additional reinforcing element can be placed above. In another embodiment the ends of each string element are not bonded to the apparatus and can be fastened in a free manner. Additional strings can be added as a standalone string laying on the outer or inner surfaces. Strings can be placed in a different direction.

In another embodiment string may consists of different sections and described as polytension element. The string having at least two sections with different elasticity, wherein the acupressure elements are attached to the sections of the strings via the rivet snaps or the setscrews, which provides a different stroke length of the acupressure elements when the strings are stretched. Due to the different tension of the string sections, the elements are pressed differently in the required place of the human's torso. Also, the amplitude of movement of the elements will be different during the stretching of the string to provide the necessary massage effect.

The acupressure elements and the strings layout can be as inside the back member or on the inner or outer surface with all possible combinations. One of the possible variations: acupressure elements are on the apparatus inner surface allowing direct contact with a skin of human's torso. The back of the apparatus is wider to maximize the number of acupressure elements. The side parts of back member are narrower to reduce the pressure on the human's ribs.

In another embodiment the acupressure cells are stitched around the acupressure elements on the back member to retain their position during the use and locate them in the necessary sectors.

The reinforcing elements of the apparatus can be of different widths, lengths and stiffness. In case of injury, reinforcing elements can be used that are not elastic and reliably hold the muscle core of the human's body, preventing it from deforming. The reinforcing elements of apparatus can be combined with the strings and be as a shape retainer of the apparatus.

Different types of fabric fasteners can be used for apparatus straps and strings, for example, hook and loop, hook and eye, rivet snaps or any other fastening element which allows to secure fastening and is known to the skilled person. The size of the apparatus can vary and fit any human's torso type according the specification.

### Brief description of the drawings

The drawings illustrate generally, by way of example, but not by way of limitation, various embodiments of the invention.
Fig. 1A is a perspective view of one embodiment of the invention.
Fig. 1B is a perspective view of another embodiment of the invention.
Fig. 3 illustrates a human torso (16) and a sagittal plane (17) of the human torso (16).
Fig. 3A is a top view of another embodiment of the invention.
Fig. 3B is perspective view of the embodiment as shown in Fig. 3A.
Figs. 4A to 4D illustrates various embodiments of acupressure elements (1).
Figs. 5A to 5F illustrates various embodiments of acupressure elements (1) as well as various embodiments on connection of the acupressure elements (1) with strings (3).
Fig. 6A is a perspective view of another embodiment of the invention comprising a back member (6). The apparatus is viewed from its outer side.
Fig. 6B is a perspective view of the embodiment as seen in Fig. 6A. The apparatus is viewed from its inner side.
Fig. 6C illustrates arrangement of the strings (3) and back strings (26) within the apparatus as seen in Figs. 6A and 6B. For ease of understanding the back member (6) is partly removed.
Fig. 6D illustrates an embodiment of the present invention where the strings (3) are threaded through a back member (6).
Fig. 6E illustrates an embodiment of the present invention where the strings (3) are threaded through a dividing cloth (9).
Fig. 6F illustrates an embodiment of the present invention where the strings (3) are within a back member (6).
Fig. 7A is a perspective view of another embodiment of the present invention.
Fig. 7B is a perspective view of another embodiment of the present invention.
Fig. 7C is a top view of another embodiment of the present invention.
Fig. 7D is a perspective view of another embodiment of the present invention.
Fig. 8 illustrates another embodiment of the present invention comprising four straps (7). The apparatus is viewed from its outer side.
Fig. 9A illustrates another embodiment of the present invention comprising four straps (7) and four string fasteners (5). The apparatus is viewed from its outer side.
Fig. 9B illustrates the apparatus as seen in Fig. 9A from its inner side.
Fig. 10A illustrates another embodiment of the present invention comprising shoulder straps (23). The apparatus is viewed from its outer side.
Fig. 10B illustrates the apparatus as seen in Fig. 10A from its inner side.
Fig. 11A illustrates another embodiment of the present invention comprising shoulder straps (23) and four straps (7). The apparatus is viewed from its outer side.
Fig. 11B illustrates the apparatus as seen in Fig. 11A from its inner side.
Fig. 12A illustrates another embodiment of the present invention from the outer side (15).
Fig. 12B illustrates the apparatus as seen in Fig. 12A from the inner side (14).

### Detailed Description of the Embodiments

The preferred embodiments of the invention are now described with reference to the figures to illustrate objectives, advantages, and efficiency of the present invention.

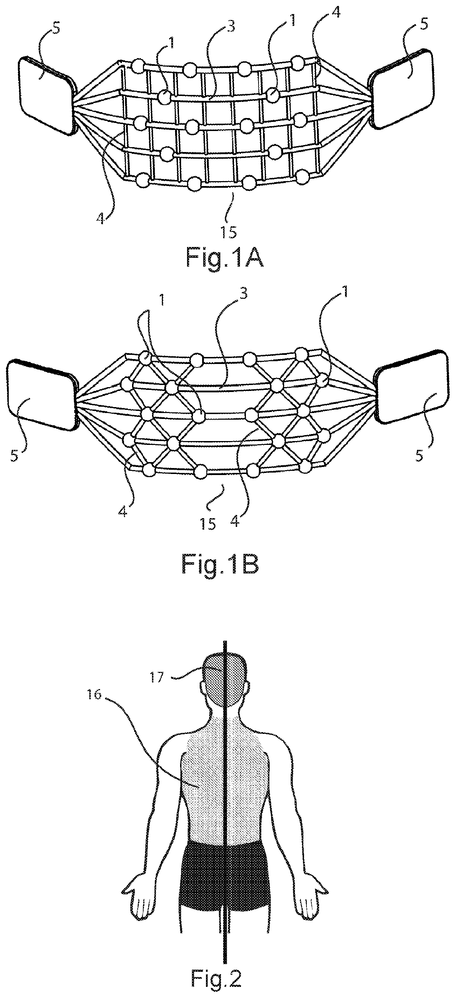

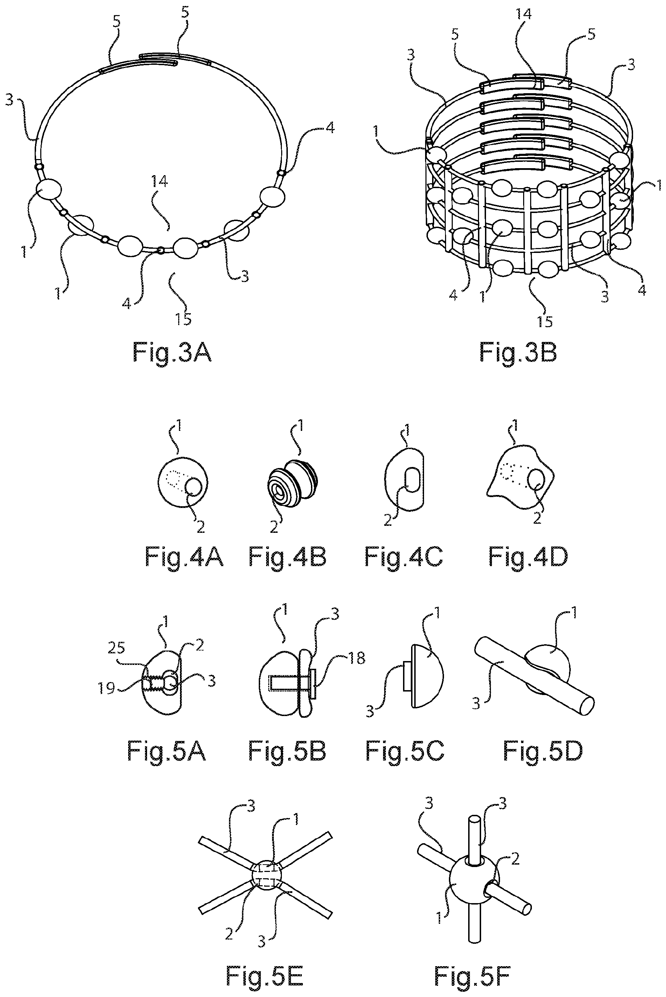

Fig. 1A is a perspective view of one embodiment of the invention which comprises essential technical features of the invention. The apparatus comprising a number of acupressure elements (1) with a distance between/from each other. The acupressure elements (1) are made of rigid material and comprising at least one canal (2) therethrough for threading at least one string (3) through the canal (2). Canals (2) are seen in Figs. 4A to 4D. The apparatus comprises five strings (3) which are an elongated elements made of a stretch material. The strings (3) wrap the human's torso (16), and thus the strings (3) press the acupressure elements (1) towards the human's torso (16). Each of the two ends of the string (3) is fastened to the string fasteners (5). The strings (3) are arranged at 90 degrees angle relative to the sagittal plane (17) of the human. Each string fastener (5) is attached to the respective string fastener (5), so that one string fastener (5) overlaps the other string fastener (5) and thus changes the distance between the ends of the strings (3), and thus changes the tension of the strings (3). The string fasteners (5) are attached to each other through hook and loop or hook and eye, or rivet snaps. The apparatus comprises eight reinforcing elements (4) which are elongated elements made from elastic and rigid material. The reinforcing elements (4) are arranged transversally in relation to the strings (3) providing not only reinforcement of the net formed by the reinforcing elements (4) and strings (3), but also providing a distance between adjacent strings (3).

Fig. 1B is a perspective view of another embodiment of the invention similar to the one shown in Fig. 1A. The embodiment in Fig. 1B differs from the embodiment in Fig. 1A in that the reinforcing elements (4) are connected to the acupressure elements (1) and are arranged diagonally in relation to the strings (3) providing not only reinforcement of the net formed by the reinforcing elements (4) and strings (3), but also providing a distance between adjacent acupressure elements (1).

Fig. 2 illustrates a human torso (16) and a sagittal plane (17) of the human torso (16). Therapeutic apparatus for a human torso (16) has an inner side (14) directed to the human's torso (16) and outer side (15) directed outside from the human's torso (16).

Fig. 3A and 3B illustrates another embodiment of the invention, wherein at each end of the string (3) separate string fastener (5) is attached.

Figs. 4A to 4D illustrates various embodiments of acupressure elements (1). Each acupressure element comprises a canal (2) for inserting the string (3). In Fig. 4A the acupressure element (1) is a sphere with the canal (2) therethrough. In Fig. 4B the acupressure element (1) has certain profile as well as the canal (2) therethrough. In Fig. 4C the acupressure element (1) has another profile as well as the canal (2) therethrough. In Fig. 4D the acupressure element (1) has irregular shape as well as the canal (2) therethrough. In present embodiments all acupressure elements (1) have at least one canal (2) for threading the string (2) or a back string (26).

Figs. 5A to 5F illustrates various embodiments of acupressure elements (1) as well as various embodiments on connection of the acupressure elements (1) with strings (3). In Fig. 5A the acupressure element (1) comprises the canal (2) as well as a threaded hole (25) arranged orthogonal in relation to the canal (2). In the threaded hole (25) a set screw (19) can be inserted (screwed) so that this set screw (19) fixes the string (3) within the canal (2), in result of which the acupressure element (1) is fixed in relation to the string (3). Instead of the set screw (19) a rivet (18) can be used as seen if Fig. 5B. Fig. 5C illustrates an embodiment of the acupressure element (1) where the string (3) is glued to the acupressure element (1). In another embodiments of the invention, the acupressure element (1) comprises a groove or recess in which the string (3) may be fixed (see Fig. 5D). In Fig. 5E the acupressure element (1) comprises two canals (2) so that in each canal (2) the separate string (3) can be inserted. Both canals do not cross of pass each other. In Fig. 5F the acupressure element (1) comprises two crossing canals (2) and the string (3) is inserted in each canal (2) of the acupressure element (1).

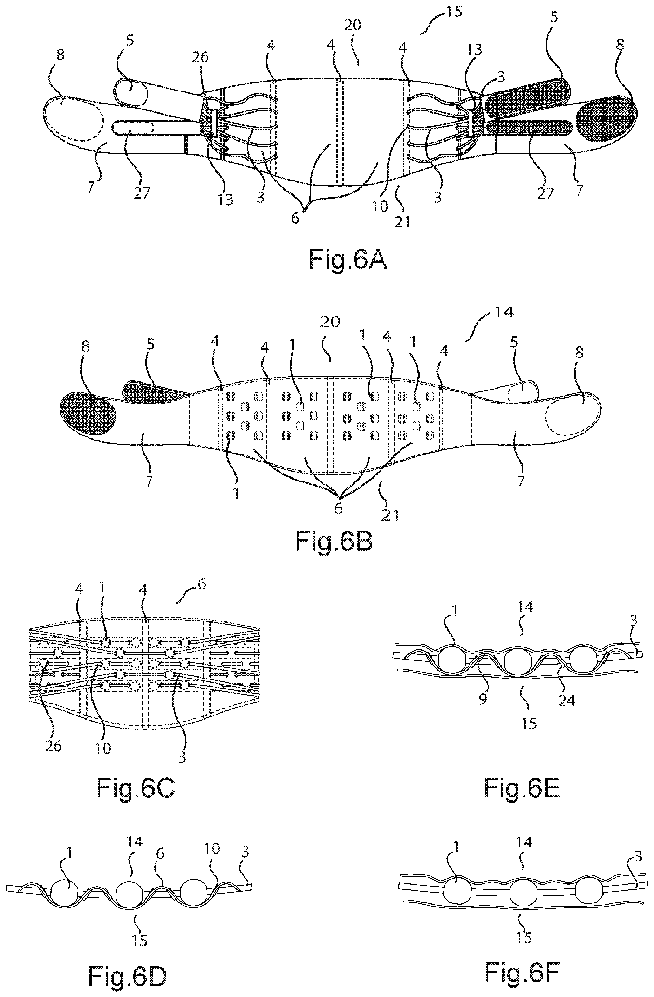

Fig. 6A and 6B illustrate another embodiment of the invention comprising a back member (6). The back member (6) is made of woven material and covers part of the back and lateral sides of human's torso. The back member (6) comprises back member eyelets (10) allowing the strings (3) to be threaded therethrough. The apparatus also comprises two straps (7), where each strap (7) is an elongated element made of elastic material. Each strap (7) is attached to the back member (6) as a continuation of the same back member (6). One strap (7) extends from the back member (6) in left direction and symmetrically to the sagittal plane (17) and another strap (7) extends from the back member (6) in the right direction, in result of which the straps (7) can wrap the human's torso. At each distal end from the back member (6) the strap (7) comprises a strap fastener (8). The reinforcing elements (4) are associated with the back member (6) so that the back member (6) extends along all length of the reinforcing elements (4). The apparatus comprising back strings (26), where one end of the back strings (26) is attached to the back member (6) and another end of the back strings (26) is attached to the back string fastener (27). The apparatus further comprises two loops (13) located on the surface of the straps (7) from outer side (15) for threading the strings (3) through it. The strings (3) wrap around the human's torso (16) and are threaded through the loops (13) for comfortable use of the therapeutic apparatus, so that even when the strings (3) are unfastened, the fasteners (5) remain in the part of the human's torso (16) accessible to the human using the therapeutic apparatus.Fig. 6C illustrates arrangement of the strings (3) and the back strings (26) within the back member (26) of the apparatus as seen in Figs. 6A and 6B. For ease of understanding the back member (6) is partly removed so that the arrangement of the strings (3) and the back strings (26) can be clearly seen.

Fig. 6D illustrates another embodiment of the present invention where the strings (3) are threaded through a back member (6) having back member eyelets.
Fig. 6E illustrates an embodiment of the present invention where the strings (3) are threaded through a dividing cloth (9) having dividing cloth eyelets (24). In addition a cloth is provided from the inner side (14) and the outer side (15) so that the clothes fully cover the dividing cloth with acupressure elements (1) and the strings (3). Fig. 6F illustrates similar embodiment of the present invention as seen in Fig 6E only without the dividing cloth.

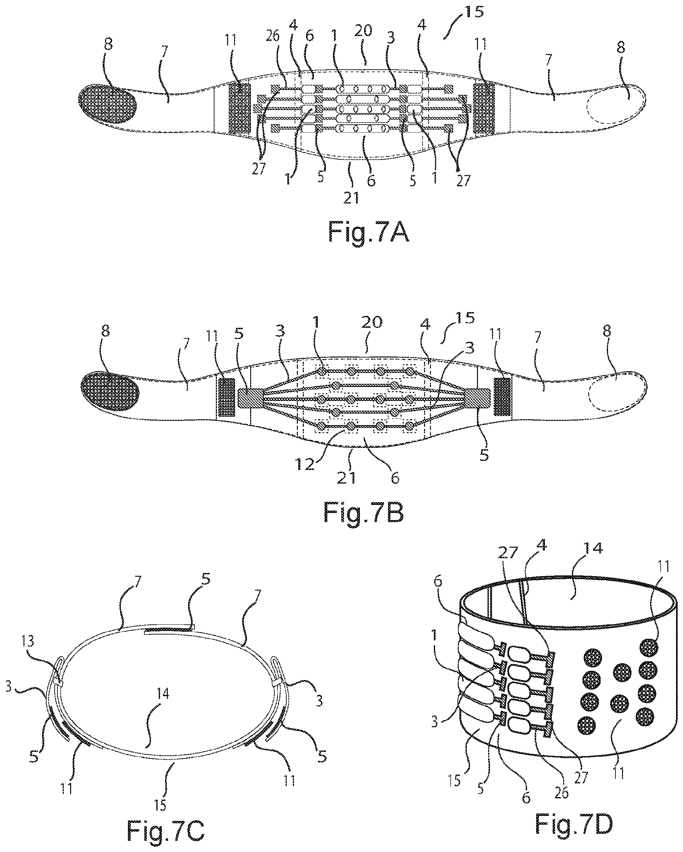

Fig. 7A illustrates another embodiment of the present invention which differs from the embodiment as seen in Fig. 7A that each string (3) and each back string (26) on each of its ends comprises its own respective string fastener (5) and back string fastener (27). Fig. 7D illustrates similar apparatus as seen in Fig. 6A where each string (3) on each of its ends comprises its own string fastener (5) and each back string (26) on each of its ends comprises its own back string fastener (27). The only difference is in a design of attachment points (11). Fig. 7B illustrates another embodiment of the present invention in which all strings (3) have common string fasteners (5). In addition, each acupressure member (1) has its own acupressure member cell (12) formed in the back member (6).

Fig. 7C illustrates another embodiment of the present invention in which the strings (3) which wrap around the human's torso (16) are threaded through the loops (13) and, having made a 180-degree bend, are fastened to the attachment point (11) at lateral or rear side of the back member (6) by the string fastener (5) or back string fastener (27).

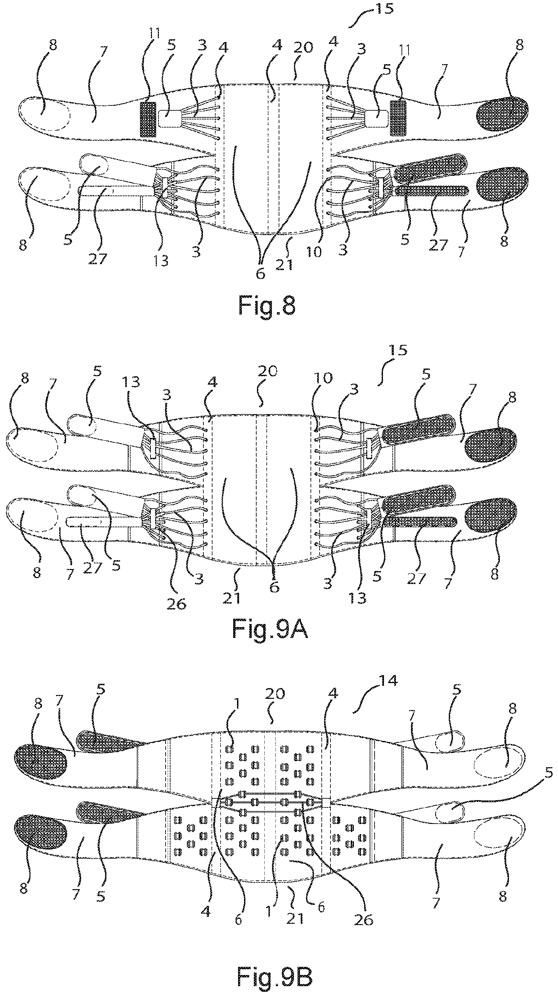

Fig. 8 illustrates another embodiment of the present invention comprising two sets of strings (3) and respective four straps (7), one set of back strings (26) and respective one set of back string straps (5).

Figs. 9A and 9B illustrate another embodiment of the present invention comprising four straps (7), two back string fasteners (27) and four string fasteners (5). The back strings (26) are located in a lower part (21) of the apparatus. Same back strings (26) are threaded through the middle portion of the back member (6).

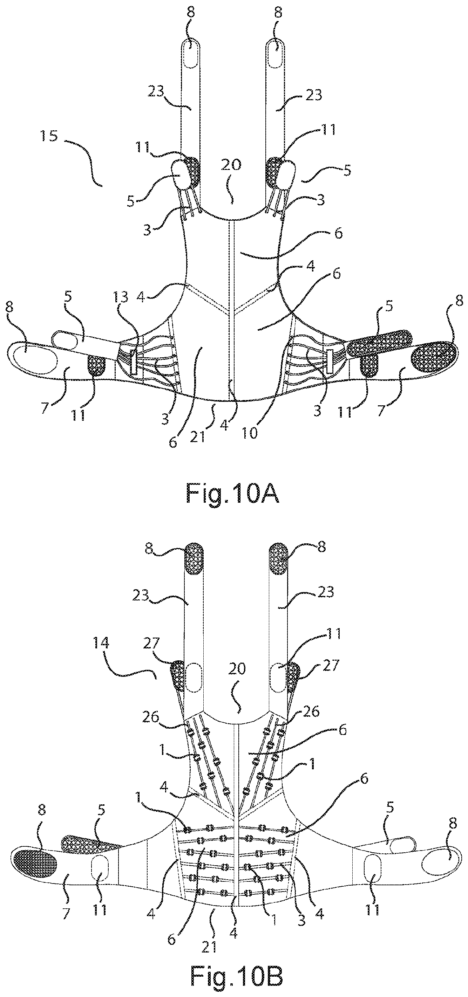

Figs. 10A and 10B illustrate another embodiment of the present invention comprising two shoulder straps (23) extending parallel and symmetrically to the sagittal plane (17) from the upper part (20) of the back member (6) and configured to wrap around the human's or wearer's shoulders. The shoulder straps (23) also comprise strap fasteners (8). In Fig. 10B arrangement of acupressure elements (1) on the back member (6) is shown.

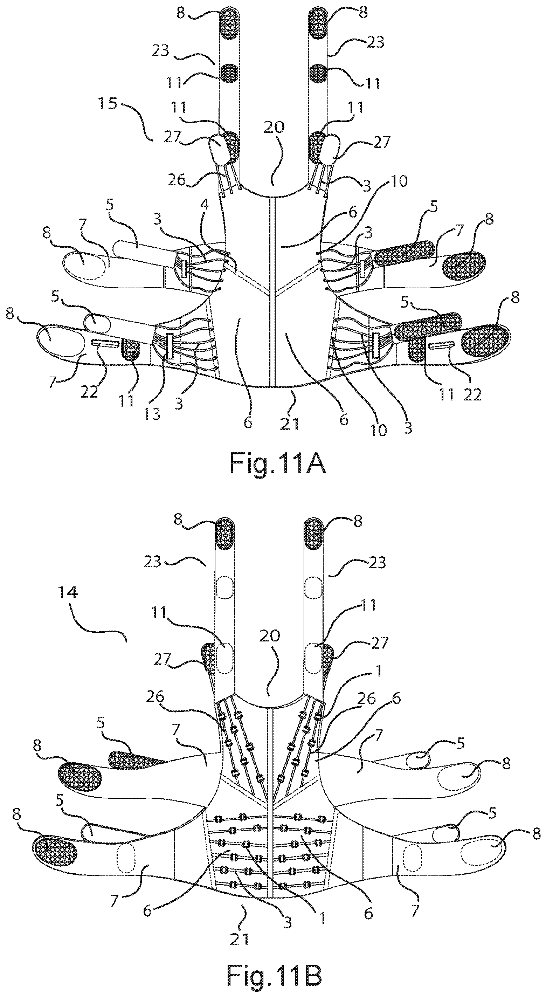

Figs. 11A and 11B illustrates another embodiment of the present invention comprising shoulder straps (23) and four straps (7). Embodiment of Figs. 11A and 11B differs from the embodiment of Figs. 10A and 10B in that it comprises additional set of strings (3), back strings (26) similar to the embodiment as seen in Figs. 8 to 9B.

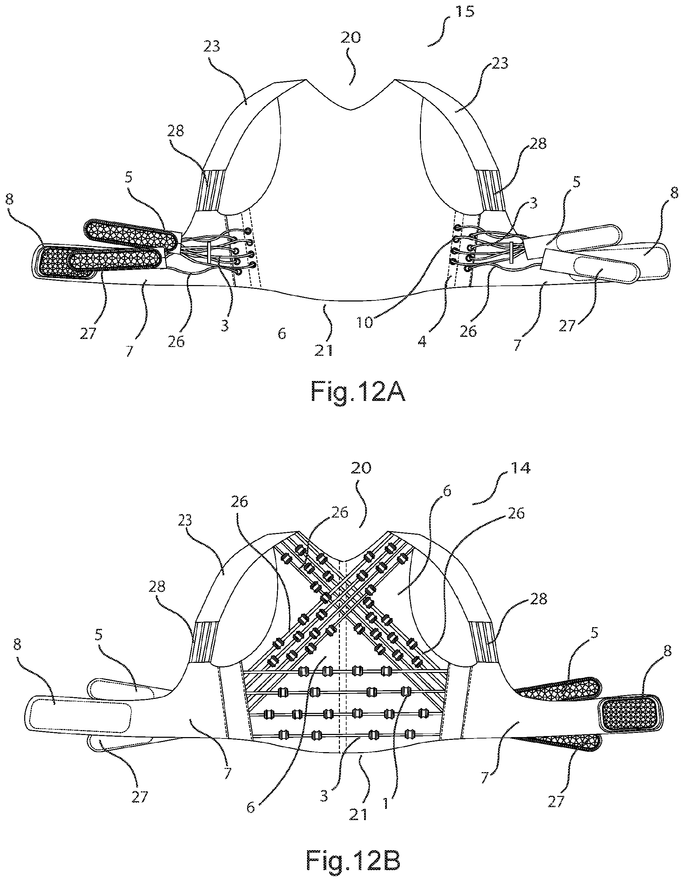

Figs. 12A and 12B illustrates another embodiment of the present invention comprising two shoulder straps (23) extending parallel and symmetrically to the sagittal plane (17) from the upper part (20) of the back member (6) and configured to wrap around the human's or wearer's shoulders while connected with the straps (7). The shoulder straps (23) are connected to the straps (7) through the elastic straps (28). One set of the strings (3) and acupressure elements (1) on them, are located in the back member perpendicular and to the sagittal plane (17) and two sets of the back strings (26) and acupressure elements (1) on them, are located in the back member diagonally to the sagittal plane (17). The back member (6) of the present invention is formed to align to the upper parts of the human's torso (16).

While the invention may be susceptible to various modifications and alternative forms, specific embodiments of which have been shown by way of example in the figures and have been described in detail herein, it should be understood that the invention is not intended to be limited to the particular forms disclosed. Rather, the invention is defined by the following claims.

### List of references

- 1.: an acupressure element;
- 2.: a canal;
- 3.: a string;
- 4.: a reinforcing element;
- 5.: a string fastener;
- 6.: a back member;
- 7.: a strap;
- 8.: a strap fastener;
- 9.: a dividing cloth;
- 10.: a back member eyelet;
- 11.: an attachment point;
- 12.: an acupressure element cell;
- 13.: a loop;
- 14.: an inner side;
- 15.: an outer side;
- 16.: a human's torso;
- 17.: a sagittal plane;
- 18.: a rivet;
- 19.: a setscrew;
- 20.: an upper part;
- 21.: a lower part;
- 22.: a strap lug;
- 23.: a shoulder strap;
- 24.: a dividing cloth eyelet;
- 25.: a threaded hole;
- 26.: a back string;
- 27.: a back string fastener; and
- 28.: an elastic strap.

## Claims

1. Therapeutic apparatus for a human torso (16), wherein in use the apparatus has an inner side (14) directed to the human's torso (16), and outer side (15) directed outside from the human's torso (16), and wherein the apparatus comprising:
- at least two acupressure elements (1) with 1 mm to 10 cm distance between/from each other, wherein the acupressure elements (1) are made of rigid material and comprising at least one canal (2) therethrough for threading at least one string (3) through the canal (2);
- at least two strings (3) which are an elongated elements made of a stretch material,
**characterized in that**
the strings (3) wrap the human's torso (16) in use, and thus press the acupressure elements (1) towards the human's torso (16); wherein the strings (3) are arranged transversally in relation to the sagittal plane (17) of the human;
wherein the therapeutic apparatus further comprises
- at least two string fasteners (5), wherein each of two ends of the string (3) is fastened to the string fastener (5); wherein one string fastener (5) is attached to another string fastener (5), so that one string fastener (5) overlaps another string fastener (5) and thus changes the distance between the ends of the strings (3), and thus changes the tension of the strings (3); and wherein the string fasteners (5) have hook and loop or hook and eye, or rivet snaps; and
- at least two reinforcing elements (4) which are elongated elements made from a material which is elastic in one direction and rigid in another direction, wherein the reinforcing elements (4) are arranged transversally in relation to the strings (3).

2. The therapeutic apparatus according to claim 1, wherein the acupressure element (1) comprises a threaded hole (25) arranged perpendicular to the canal (2) for a set screw (19), allowing the set screw (19) to hold the string (3) within the canal (2) thereby holding the acupressure element (1) from sliding along the string (3).

3. The therapeutic apparatus according to claim 1 or 2, wherein one of the two ends of the reinforcing element (4) is attached to one string (3), and another end of the reinforcing element (4) is attached to another string (3), thereby providing a distance between the strings (3) from 1 cm to 10 cm and wherein interconnected strings (3) and reinforcing elements (4) form a flexible net.

4. The therapeutic apparatus according to claim 1 or 2, further comprising:
- a back member (6) which is made of woven material at least from one layer of fabric and, in use, covers part of the back and lateral sides of human's torso, wherein the back member (6) comprises back member eyelets (10) allowing the strings (3) to be threaded therethrough;
- at least one strap (7) which is an elongated element made of elastic material, which are continuation of the back member (6), extended from the back member (6) in left direction and symmetrically to the sagittal plane (17) the same numbers of straps (7) extended from the back member (6) in the right direction and straps (7), in use, wrap the human's torso; at each distal end from the back member (6) the strap (7) comprises a strap fastener (8);
wherein the reinforcing elements (4) are associated with the back member (6) so that the back member (6) extends along all length of the reinforcing elements (4).

5. The therapeutic apparatus according to claim 1, 2 or 4, wherein the back member (6) comprises a dividing cloth (9) with dividing cloth eyelets (24) through which the strings (3) are threaded as follows: the string (3) is threaded from the outer side (15) through the dividing cloth eyelet (24) of the dividing cloth (9), then threaded through the acupressure element (1), then threaded through the dividing cloth eyelet (24) of the dividing cloth (9) from the inner side (14), then threaded through the dividing cloth eyelet (24) from the outer side (15) of dividing cloth (9) and so on alternates with at least two acupressure elements (1).

6. The therapeutic apparatus according to any of claim 1, 2, 4 and 5, wherein
- the back member (6) comprises at least two layers of cloth, between which the acupressure elements (1), the reinforcing elements (4), the strings (3) and the dividing cloth (9) are positioned;
- the therapeutic apparatus comprising a back string (26), where one end of the back string (26) is attached to the back member (6) and another end of the back string (26) is attached to the back string fastener (27); and
- at least two loops (13) located on the surface of the straps (7) from outer side (15) for threading the strings (3) through it, and wherein the strings (3), in use, wrap around the human's torso (16) and are threaded through the loops (13) for comfortable use of the therapeutic apparatus, so that even when the strings (3) are unfastened, the fasteners (5) remain in the part of the torso (16) accessible to the human using the therapeutic apparatus.

7. The therapeutic apparatus according to any of claim 1, 2, or 4 to 6, wherein at least one of the strings (3) or the back strings (26) has at least two section with different elasticity, wherein the acupressure elements (1) are attached to at least two sections of the strings (3) or the back strings (26) via the rivets (18) or the setscrews (19), in result of which a different stroke length of the acupressure elements (1) is provided when the strings (3) or the back strings (26) are stretched.

8. The therapeutic apparatus according to any of claim 1, 2, or 4 to 7, further comprising at least two attachment points (11) for attachment of the strap fasteners (8) or the string fastener (5), which are located on the surface of straps (7) or on a surface of the back member (6) symmetrically to the sagittal plane (17).

9. The therapeutic apparatus according to any of claim 1 to 8, wherein the strings (3) and back strings (26) which in use wrap around the human's torso (16) are threaded through the loops (13) and, having made a 180-degree bend, are fastened to the attachment point at lateral or rear side of the back member (6) by the string fastener (5) or back string fastener (27).

10. The therapeutic apparatus, according to any of claim 1 to 9, wherein at least one back string (26) one end is fixed to the back member (6) in a one place, and the other end of the back string (26) is fixed to the back member (6) in the other place, thus the back strings (26) are stretched with the back member (6) when the straps (7) are fastened around the human's torso (16).

11. The therapeutic apparatus according to any of claim 1 to 10, further comprises at least two cells (12) for acupressure elements (1) in the back member (6), formed by stitching together at least two fabric layers of the back member (6) around the acupressure element (1) with the string (3) threaded through the acupressure element (1); and wherein at least two acupressure elements (1) are located inside the cells (12) for acupressure elements (1); thus, the cells (12) for acupressure elements (1) limits the length of dislocation of acupressure element (1) at length equal to acupressure element (1) length, which provides a massage effect when the human moves using the apparatus.

12. The therapeutic apparatus, according to any of claim 1 to 11, further comprises a pair of shoulder straps (23) extending parallel and symmetrically to the sagittal plane (17) from the upper part (20) of the back member (6), and, in use, wrap around the human's shoulders and, using strap fasteners (8) at the ends of the shoulder straps (23), are attached to the attachment point (11) on the straps (7).

13. The therapeutic apparatus, according to any of claim 1 to 12, further comprises at least two strap lugs (22) which located on the surface of the strap (7); and a pair of the shoulder straps (23) extending parallel and symmetrically to the sagittal plane (17) from the upper part (20) of the back member (6), wrap in use around the user's shoulders, are threaded through the strap lugs (22) on the straps (7) and, having made a 180-degree bend, are fastened on the attachment point (11) on the shoulder strap (23) by the strap fasteners (8) on the shoulder straps (23).

14. The therapeutic apparatus according to any of claim 1 to 13, wherein a pair of shoulder straps (23) extend parallel and symmetrically to the sagittal plane from the upper part (20) of the back member (6) and, in use, wrap around the human's shoulders, and wherein shoulder straps (23) are attached to the straps (7) by the at least one elastic band. (28).

## Patentansprüche

1. Therapeutische Vorrichtung für einen menschlichen Oberkörper (16), wobei die Vorrichtung eine dem menschlichen Oberkörper (16) zugewandte Innenseite (14) und eine vom menschlichen Oberkörper (16) nach außen gerichtete Außenseite (15) aufweist, und wobei die Vorrichtung umfasst:
- mindestens zwei Akupressurelemente (1) mit einem Abstand von 1 mm bis 10 cm zueinander, wobei die Akupressurelemente (1) aus einem starren Material bestehen und mindestens einen durchgehenden Kanal (2) zum Durchziehen mindestens einer Schnur (3) durch den Kanal (2) aufweisen;
- mindestens zwei Schnüre (3), die längliche Elemente aus einem dehnbaren Material sind,
**dadurch gekennzeichnet, dass**
die Schnüre (3) bei der Anwendung den Oberkörper (16) des Menschen umschlingen und dadurch die Akupressurelemente (1) gegen den Oberkörper (16) des Menschen drücken;
wobei die Schnüre (3) quer zur Sagittalebene (17) des Menschen angeordnet sind;
wobei die therapeutische Vorrichtung ferner umfasst
- mindestens zwei Schnurbefestigungen (5), wobei jedes der beiden Enden der Schnur (3) an der Schnurbefestigung (5) befestigt ist; wobei eine Schnurbefestigung (5) an einer anderen Schnurbefestigung (5) angebracht ist, so dass eine Schnurbefestigung (5) eine andere Schnurbefestigung (5) überlappt und dadurch den Abstand zwischen den Enden der Schnüre (3) verändert, und dadurch die Spannung der Schnüre (3) verändert; und wobei die Schnurbefestiger (5) Klettverschlüsse oder Haken- und Ösenverschlüsse oder Druckknöpfe aufweisen; und
- mindestens zwei Verstärkungselemente (4), bei denen es sich um längliche Elemente aus einem Material handelt, das in einer Richtung elastisch und in einer anderen Richtung starr ist, wobei die Verstärkungselemente (4) quer zu den Saiten (3) angeordnet sind.

2. Therapeutische Vorrichtung nach Anspruch 1, wobei das Akupressurelement (1) ein senkrecht zum Kanal (2) angeordnetes Gewindeloch (25) für eine Stellschraube (19) aufweist, wodurch die Stellschraube (19) die Schnur (3) innerhalb des Kanals (2) festhalten kann und somit das Akupressurelement (1) daran hindert, entlang der Schnur (3) zu gleiten.

3. Therapeutische Vorrichtung nach Anspruch 1 oder 2, wobei eines der beiden Enden des Verstärkungselements (4) an einer Schnur (3) befestigt ist und ein anderes Ende des Verstärkungselements (4) an einer anderen Schnur (3) befestigt ist, wodurch ein Abstand zwischen den Schnüren (3) von 1 cm bis 10 cm geschaffen wird, und wobei die miteinander verbundenen Schnüre (3) und Verstärkungselemente (4) ein flexibles Netz bilden.

4. Therapeutische Vorrichtung nach Anspruch 1 oder 2, ferner umfassend:
- ein Rückenteil (6), das aus gewebtem Material aus mindestens einer Gewebeschicht besteht und im Gebrauch einen Teil des Rückens und der Seiten des menschlichen Oberkörpers bedeckt, wobei das Rückenteil (6) Ösen (10) aufweist, durch die die Schnüre (3) hindurchgeführt werden können;
- mindestens einen Gurt (7), der ein längliches Element aus elastischem Material ist, das eine Fortsetzung des Rückenteils (6) darstellt, sich vom Rückenteil (6) in linker Richtung erstreckt und symmetrisch zur Sagittalebene (17) angeordnet ist, wobei die gleiche Anzahl an Gurten (7) sich vom Rückenteil (6) in rechter Richtung erstreckt und die Gurte (7) im Gebrauch den menschlichen Oberkörper umschlingen; an jedem vom Rückenelement (6) entfernten Ende weist der Gurt (7) einen Gurtschluss (8) auf;
wobei die Verstärkungselemente (4) mit dem Rückenteil (6) verbunden sind, sodass sich das Rückenteil (6) über die gesamte Länge der Verstärkungselemente (4) erstreckt.

5. Therapeutische Vorrichtung nach Anspruch 1, 2 oder 4, wobei das Rückenteil (6) ein Trenntuch (9) mit Trenntuchösen (24) umfasst, durch die die Schnüre (3) wie folgt geführt werden: Die Schnur (3) wird von der Außenseite (15) durch die Trenntuchöse (24) des Trenntuchs (9) geführt, dann durch das Akupressurelement (1) geführt, dann von der Innenseite (14) durch die Trenngewebeöse (24) des Trenngewebes (9) geführt, dann von der Außenseite (15) des Trenngewebes (9) durch die Trenngewebeöse (24) geführt und so weiter, abwechselnd mit mindestens zwei Akupressurelementen (1).

6. Therapeutische Vorrichtung nach einem der Ansprüche 1, 2, 4 und 5, wobei
- das Rückenteil (6) mindestens zwei Stofflagen umfasst, zwischen denen die Akupressurelemente (1), die Verstärkungselemente (4), die Schnüre (3) und das Trenntuch (9) angeordnet sind;
- die therapeutische Vorrichtung eine Rückenschnur (26) umfasst, wobei ein Ende der Rückenschnur (26) am Rückenteil (6) und ein anderes Ende der Rückenschnur (26) am Rückenschnurverschluss (27) befestigt ist; und
- mindestens zwei Schlaufen (13) an der Oberfläche der Gurte (7) von der Außenseite (15) aus zum Durchziehen der Schnüre (3) vorgesehen sind, wobei die Schnüre (3) im Gebrauch um den Oberkörper (16) des Benutzers gewickelt und durch die Schlaufen (13) geführt werden, um eine bequeme Verwendung der therapeutischen Vorrichtung zu gewährleisten, sodass selbst wenn die Schnüre (3) gelöst sind, die Befestigungselemente (5) in dem für den Benutzer der therapeutischen Vorrichtung zugänglichen Bereich des Oberkörpers (16) verbleiben.

7. Therapeutische Vorrichtung nach einem der Ansprüche 1, 2 oder 4 bis 6, wobei mindestens eine der Schnüre (3) oder der Rückenschnüre (26) mindestens zwei Abschnitte mit unterschiedlicher Elastizität aufweist, wobei die Akupressurelemente (1) über die Nieten (18) oder die Stellschrauben (19) an mindestens zwei Abschnitten der Schnüre (3) oder der Rückenschnüre (26) befestigt sind, wodurch beim Dehnen der Schnüre (3) oder der Rückenschnüre (26) eine unterschiedliche Hublänge der Akupressurelemente (1) bereitgestellt wird.

8. Die therapeutische Vorrichtung gemäß einem der Ansprüche 1, 2 oder 4 bis 7, ferner umfassend mindestens zwei Befestigungspunkte (11) zur Befestigung der Gurtverschlüsse (8) oder des Schnurverschlusses (5), die sich auf der Oberfläche der Gurte (7) oder auf einer Oberfläche des Rückenteils (6) symmetrisch zur Sagittalebene (17) befinden.

9. Therapeutische Vorrichtung nach einem der Ansprüche 1 bis 8, wobei die Schnüre (3) und Rückenschnüre (26), die im Gebrauch den menschlichen Oberkörper (16) umschlingen, durch die Schlaufen (13) geführt werden und nach einer 180-Grad-Biegung mit dem Schnurverschluss (5) oder dem Rückenschnurverschluss (27) an der seitlichen oder hinteren Seite des Rückenteils (6) befestigt werden.

10. Therapeutische Vorrichtung nach einem der Ansprüche 1 bis 9, wobei mindestens eine Rückenschnur (26) an einem Ende an einer Stelle am Rückenteil (6) befestigt ist und das andere Ende der Rückenschnur (26) an einer anderen Stelle am Rückenteil (6) befestigt ist, wodurch die Rückenschnüre (26) mit dem Rückenteil (6) gespannt werden, wenn die Gurte (7) um den Oberkörper (16) der Person befestigt werden.

11. Die therapeutische Vorrichtung nach einem der Ansprüche 1 bis 10 umfasst ferner mindestens zwei Kammern (12) für Akupressurelemente (1) im Rückenteil (6), die dadurch gebildet werden, dass mindestens zwei Gewebelagen des Rückenteils (6) um das Akupressurelement (1) herum zusammengenäht werden, wobei der Faden (3) durch das Akupressurelement (1) geführt ist; wobei sich mindestens zwei Akupressurelemente (1) innerhalb der Zellen (12) für Akupressurelemente (1) befinden; somit begrenzen die Zellen (12) für Akupressurelemente (1) die Auslenkungslänge des Akupressurelements (1) auf eine Länge, die der Länge des Akupressurelements (1) entspricht, was einen Massageeffekt bewirkt, wenn sich der Mensch unter Verwendung der Vorrichtung bewegt.

12. Die therapeutische Vorrichtung gemäß einem der Ansprüche 1 bis 11 umfasst ferner ein Paar Schultergurte (23), die sich parallel und symmetrisch zur Sagittalebene (17) vom oberen Teil (20) des Rückenelements (6) erstrecken, und die im Gebrauch die Schultern der Person umschlingen und mittels Riemenverschlüssen (8) an den Enden der Schulterriemen (23) an dem Befestigungspunkt (11) an den Riemen (7) befestigt werden.

13. Die therapeutische Vorrichtung gemäß einem der Ansprüche 1 bis 12 umfasst ferner
mindestens zwei Riemenösen (22), die sich auf der Oberfläche des Riemens (7) befinden; und
ein Paar der Schultergurte (23), die sich parallel und symmetrisch zur Sagittalebene (17) vom oberen Teil (20) des Rückenteils (6) erstrecken, die im Gebrauch um die Schultern des Benutzers gelegt werden, durch die Riemenösen (22) an den Riemen (7) geführt werden und, nachdem sie eine 180-Grad-Biegung gebildet haben, am Befestigungspunkt (11) am Schulterriemen (23) durch die Riemenverschlüsse (8) an den Schulterriemen (23) befestigt werden.

14. Therapeutische Vorrichtung nach einem der Ansprüche 1 bis 13, wobei sich ein Paar Schultergurte (23) parallel und symmetrisch zur Sagittalebene vom oberen Teil (20) des Rückenelements (6) aus erstreckt und im Gebrauch die Schultern der Person umschließt, und wobei die Schultergurte (23) über das mindestens eine elastische Band (28) an den Gurten (7) befestigt sind.

## Revendications

1. Appareil thérapeutique destiné au torse humain (16), dans lequel l'appareil présente une face interne (14) tournée vers le torse humain (16) et une face externe (15) tournée vers l'extérieur du torse humain (16), et dans lequel l'appareil comprend:
- au moins deux éléments d'acupression (1) espacés de 1 mm à 10 cm les uns des autres, lesdits éléments d'acupression (1) étant réalisés en un matériau rigide et comportant au moins un canal (2) les traversant pour permettre le passage d'au moins une cordelette (3) à travers ledit canal (2);
- au moins deux cordons (3) qui sont des éléments allongés en matériau extensible,
**caractérisé en ce que**
les cordons (3) s'enroulent autour du torse (16) de l'utilisateur et pressent ainsi les éléments d'acupression (1) contre le torse (16) de l'utilisateur ; les cordons (3) étant disposés transversalement par rapport au plan sagittal (17) de l'utilisateur;
dans lequel l'appareil thérapeutique comprend en outre
- au moins deux attaches de cordon (5), chacune des deux extrémités du cordon (3) étant fixée à une attache de cordon (5) ; une attache de cordon (5) étant fixée à une autre attache de cordon (5), de sorte qu'une attache de cordon (5) recouvre une autre attache de cordon (5) et modifie ainsi la distance entre les extrémités des cordons (3), et modifie ainsi la tension des cordons (3) ; et dans lequel les attaches de cordon (5) comportent des fermetures à crochets et boucles, à agrafes ou à rivets; et
- au moins deux éléments de renfort (4) qui sont des éléments allongés réalisés dans un matériau élastique dans une direction et rigide dans une autre direction, les éléments de renfort (4) étant disposés transversalement par rapport aux cordes (3).

2. Appareil thérapeutique selon la revendication 1, dans lequel l'élément d'acupression (1) comprend un trou fileté (25) disposé perpendiculairement au canal (2) pour une vis de réglage (19), permettant à la vis de réglage (19) de maintenir le cordon (3) à l'intérieur du canal (2), empêchant ainsi l'élément d'acupression (1) de glisser le long du cordon (3).

3. Appareil thérapeutique selon la revendication 1 ou 2, dans lequel l'une des deux extrémités de l'élément de renfort (4) est fixée à un cordon (3), et l'autre extrémité de l'élément de renfort (4) est fixée à un autre cordon (3), créant ainsi une distance entre les cordons (3) comprise entre 1 cm et 10 cm, et dans lequel les cordons (3) et les éléments de renfort (4) interconnectés forment un filet flexible.

4. Appareil thérapeutique selon la revendication 1 ou 2, comprenant en outre :
- un élément dorsal (6) qui est constitué d'un matériau tissé comprenant au moins une couche de tissu et qui, lors de l'utilisation, recouvre une partie du dos et des côtés latéraux du torse humain, dans lequel l'élément dorsal (6) comprend des œillets (10) permettant de faire passer les cordons (3) à travers ceux-ci;
- au moins une sangle (7) qui est un élément allongé en matériau élastique, constituant le prolongement de l'élément dorsal (6), s'étendant depuis l'élément dorsal (6) vers la gauche et symétriquement par rapport au plan sagittal (17) ; le même nombre de sangles (7) s'étendant depuis l'élément dorsal (6) vers la droite ; et les sangles (7), lors de l'utilisation, enveloppent le torse de l'être humain ; à chaque extrémité distale de l'élément dorsal (6), la sangle (7) comprend une attache de sangle (8);
dans lequel les éléments de renfort (4) sont associés à l'élément dorsal (6) de telle sorte que l'élément dorsal (6) s'étende sur toute la longueur des éléments de renfort (4).

5. Appareil thérapeutique selon la revendication 1, 2 ou 4, dans lequel l'élément dorsal (6) comprend une toile de séparation (9) dotée d'œillets (24) à travers lesquels les cordons (3) sont enfilés comme suit : le cordon (3) est enfilé depuis le côté extérieur (15) à travers l'œillet (24) de la toile de séparation (9), puis à travers l'élément d'acupression (1), puis à travers l'œillet (24) de la toile de séparation (9) depuis le côté intérieur (14), puis à travers l'œillet (24) de la toile de séparation (9) depuis le côté extérieur (15) de la toile de séparation (9), et ainsi de suite en alternance avec au moins deux éléments d'acupression (1).

6. Appareil thérapeutique selon l'une quelconque des revendications 1, 2, 4 et 5, dans lequel
- l'élément dorsal (6) comprend au moins deux couches de tissu, entre lesquelles sont positionnés les éléments d'acupression (1), les éléments de renfort (4), les cordons (3) et le tissu de séparation (9);
- l'appareil thérapeutique comprend un cordon dorsal (26), dont une extrémité est fixée à l'élément dorsal (6) et l'autre extrémité est fixée à la fixation de cordon dorsal (27); et
- au moins deux boucles (13) situées sur la surface des sangles (7) depuis le côté extérieur (15) pour y enfiler les cordons (3), et dans lequel les cordons (3), lors de l'utilisation, s'enroulent autour du torse (16) de l'utilisateur et sont enfilées à travers les boucles (13) pour une utilisation confortable de l'appareil thérapeutique, de sorte que même lorsque les cordons (3) sont détachés, les attaches (5) restent dans la partie du torse (16) accessible à l'utilisateur de l'appareil thérapeutique.

7. Appareil thérapeutique selon l'une quelconque des revendications 1, 2 ou 4 à 6, dans lequel au moins l'une des cordes (3) ou des cordes arrière (26) comporte au moins deux sections présentant une élasticité différente, dans lequel les éléments d'acupression (1) sont fixés à au moins deux sections des cordes (3) ou des cordes arrière (26) au moyen des rivets (18) ou des vis de réglage (19), ce qui permet d'obtenir une longueur de course différente des éléments d'acupression (1) lorsque les cordons (3) ou les cordons arrière (26) sont tendus.

8. L'appareil thérapeutique selon l'une quelconque des revendications 1, 2 ou 4 à 7, comprenant en outre au moins deux points de fixation (11) pour la fixation des attaches de sangles (8) ou de l'attache de cordon (5), qui sont situés sur la surface des sangles (7) ou sur une surface de l'élément dorsal (6) symétriquement par rapport au plan sagittal (17).

9. Appareil thérapeutique selon l'une quelconque des revendications 1 à 8, dans lequel les cordons (3) et les cordons arrière (26) qui, lors de l'utilisation, s'enroulent autour du torse (16) de l'utilisateur sont enfilés à travers les boucles (13) et, après avoir effectué un coude à 180 degrés, sont fixées au point d'attache situé sur le côté latéral ou arrière de l'élément dorsal (6) par l'attache de cordon (5) ou l'attache de cordon dorsal (27).

10. Appareil thérapeutique selon l'une quelconque des revendications 1 à 9, dans lequel au moins une corde dorsale (26) a une extrémité fixée à l'élément dorsal (6) en un seul endroit, et l'autre extrémité de la sangle dorsale (26) est fixée à l'élément dorsal (6) en un autre emplacement, de sorte que les sangles dorsales (26) sont tendues avec l'élément dorsal (6) lorsque les sangles (7) sont fixées autour du torse (16) de l'utilisateur.

11. L'appareil thérapeutique selon l'une quelconque des revendications 1 à 10 comprend en outre au moins deux alvéoles (12) destinées à recevoir des éléments d'acupression (1) dans l'élément dorsal (6), formées en cousant ensemble au moins deux couches de tissu de l'élément dorsal (6) autour de l'élément d'acupression (1) avec le fil (3) enfilé à travers l'élément d'acupression (1) ; et dans lequel au moins deux éléments d'acupression (1) sont situés à l'intérieur des alvéoles (12) destinées aux éléments d'acupression (1) ; ainsi, les alvéoles (12) destinées aux éléments d'acupression (1) limitent la longueur de déplacement de l'élément d'acupression (1) à une longueur égale à celle de l'élément d'acupression (1), ce qui procure un effet de massage lorsque l'utilisateur se déplace en utilisant l'appareil.

12. L'appareil thérapeutique, selon l'une quelconque des revendications 1 à 11, comprend en outre une paire de bretelles (23) s'étendant parallèlement et symétriquement au plan sagittal (17) à partir de la partie supérieure (20) de l'élément dorsal (6), et qui, lors de l'utilisation, s'enroulent autour des épaules de l'utilisateur et, à l'aide de fixations de sangles (8) situées aux extrémités des bretelles (23), sont fixées au point d'attache (11) sur les sangles (7).

13. L'appareil thérapeutique, selon l'une quelconque des revendications 1 à 12, comprend en outre
au moins deux pattes de sangle (22) situées sur la surface de la sangle (7); et
une paire de bretelles (23) s'étendant parallèlement et symétriquement au plan sagittal (17) à partir de la partie supérieure (20) de l'élément dorsal (6), s'enroulent, lors de l'utilisation, autour des épaules de l'utilisateur, sont enfilées à travers les pattes de fixation (22) sur les sangles (7) et, après avoir effectué un coude à 180 degrés, sont fixées au point de fixation (11) sur la sangle d'épaule (23) par les attaches de sangle (8) sur les sangles d'épaule (23).

14. Dispositif thérapeutique selon l'une quelconque des revendications 1 à 13, dans lequel une paire de bretelles (23) s'étend parallèlement et symétriquement au plan sagittal à partir de la partie supérieure (20) de l'élément dorsal (6) et, lors de l'utilisation, s'enroule autour des épaules de l'utilisateur, et dans lequel les bretelles (23) sont fixées aux sangles (7) par au moins une bande élastique (28).
